# EUROPEAN PATENT SPECIFICATION

(11) **EP 3 231 787 B1**
(45) Date of publication and mention of the grant of the patent: **02.12.2020**
(21) Application number: 15867089.3
(22) Date of filing: 28.07.2015
(51) Int. Cl.: C07C 41/18, C07C 43/12, C07C 51/58, C07C 59/135, C08G 65/00, C07B 61/00

(54) **METHOD FOR PRODUCING FLUOROPOLYETHER**
VERFAHREN ZUR HERSTELLUNG VON FLUORPOLYETHER
MÉTHODE DE PRODUCTION D'UN POLYÉTHER FLUORÉ

(30) Priority: 12.12.2014 JP 2014251666
(43) Date of publication of application: 18.10.2017
(73) Proprietor: MORESCO CORPORATION, Kobe-shi Hyogo 650-0047 (JP)
(72) Inventor: SHIMIZU, Tsuyoshi, Kobe-shi Hyogo 650-0047 (JP)
(74) Representative: Winter, Brandl, Fürniss, Hübner, Röss, Kaiser, Polte - Partnerschaft mbB
(86) International application number: PCT/JP2015/071897
(87) International publication number: WO 2016/092900

(56) References cited:
- WO-A1-00/56694
- WO-A1-2009/008380
- JP-A- H1 029 955
- MURATA K ET AL: "THE THERMAL DECOMPOSITION OF PERFLUOROESTERS", JOURNAL OF THE AMERICAN CHEMICAL SOCIETY, AMERICAN CHEMICAL SOCIETY, US, vol. 120, no. 28, 1 January 1998 (1998-01-01), XP002929013, ISSN: 0002-7863, DOI: 10.1021/JA980727B
- VIACHESLAV A. PETROV: "Reaction of Boron Triflate with Polyfluoroolefins. Synthesis of Polyfluorinated Allyl Trifluoromethanesulfonates +", THE JOURNAL OF ORGANIC CHEMISTRY, vol. 63, no. 9, 1 May 1998 (1998-05-01), pages 2988-2992, XP055444477, US ISSN: 0022-3263, DOI: 10.1021/jo972166x
- BEHR,F.E. ET AL.: 'A Lewis acid catalytic process for preparing fluorocarboxylic acid halides' JOURNAL OF FLUORINE CHEMISTRY vol. 112, no. 2, 2001, pages 369 - 372, XP004312738

## Description

### TECHNICAL FIELD

The present invention relates to a process for preparing a fluoropolyether by cleavage of a fluoropolyether having a hydroxyl group, the former fluoropolyether being lower than the latter fluoropolyether in molecular weight.

### BACKGROUND ART

A fluoropolyether is obtained, for example, by photooxidation of a perfluoroolefin, ring-opening polymerization of a partially fluorinated oxetane compound, fluorination of a hydrogenated polyalkylene compound (GB 1104482 and JP 1985-202122A). In these methods, it is generally known that a final product having too much molecular weight is obtained and use thereof is practically restricted. In practice, these high-molecular weight product was applied only to the limited use. Among very interesting applications, there is an application in electric or electronic fields in which a low-molecular weight fluoropolyether is required.

A process is known in which a fluoropolyether having no hydroxyl group is cleaved using a Lewis acid to obtain a low-molecular weight fluoropolyether (US 4720527).

JP H10 29955 A discloses a cracking process applied to perfluoropolyethers wherein the ether bond is cleaved under heat and in the presence of a Lewis acid.

A process for the Lewis acid and heat mediated conversion of perfluoroalkanesulfonide fluorides or chlorides into perfluoroalkan acid fluorides is known (BEHR,F.E. ET AL., J. Fluor. Chem., vol. 112, no. 2, 2001, pages 369-372).

WO 00/56694 A1 and WO 2009/008380 A1 each discloses a process for preparing a fluoropolyether by decomposing a trifluoroacetate in the presence of a Lewis acid.

A process for the thermal decomposition of trifluoroacetate fluoropolyethers to provide the corresponding fluoropolyether acid fluoride is known (MURATA K ET AL., J. Am. Chem. Soc., vol. 120, no. 28, 1998).

A process for the Lewis acid mediated thermal decomposition of triflate substituted fluoropolymers to result in the corresponding acid fluorides is known (VIACHESLAV A. PETROV, J. Org. Chem., vol. 63, no. 9, 1998, pages 2988-2992).

However, a process is not known in which a fluoropolyether having hydroxyl group is cleaved to obtain a low-molecular weight fluoropolyether, since the fluoropolyether deactivates a Lewis acid.

### SUMMARY OF THE INVENTION

### PROBLEM TO BE SOLVED BY THE INVENTION

An object of the present invention is to provide a process for preparing a mixture of low-molecular weight fluoropolyether and acid fluoride by cleavage of a fluoropolyether having a hydroxyl group.

### MEANS FOR SOLVING THE PROBLEM OF THE INVENTION

The present invention provides a process according to claim 1 or 2 for preparing a mixture of low-molecular weight fluoropolyether and acid fluoride by decomposing with heating a triflate or trifluoroacetate of a fluoropolyether having a hydroxyl group in the presence of a Lewis acid.

### EFFECT OF THE INVENTION

In the present invention, it is possible to prepare a mixture of a fluoropolyether having a molecular weight lower than a starting fluoropolyether and an acid fluoride by modifying a hydroxyl group of the starting fluoropolyether to a triflate or trifluoroacetate and cleaving the fluoropolyether with heating in the presence of a Lewis acid.

### EMBODIMENT OF PRACTICING THE INVENTION

The fluoropolyether having a hydroxyl group is at least one selected among:
A-(CF₂CF₂O)x(CF₂O)yCF₂CH₂OH HOCH₂CF₂O(CF₂CF₂O)x(CF₂O)yCF₂CH₂OH
A-(CF₂CF₂CF₂O)zCF₂CF₂CH₂OH HOCH₂CF₂CF₂O(CF₂CF₂CF₂O)zCF₂CF₂CH₂OH
A-(CF₂CF₂CF₂CF₂O)nCF₂CF₂CF₂CH₂OH HOCH₂CF₂CF₂CF₂O(CF₂CF₂CF₂CF₂O)nCF₂CF₂CF₂CH₂OH,
A is fluoroalkoxy, x, y, z and n are a real number of 1 to 100.

Examples of fluoroalkoxy groups of A are fluoroalkoxy groups having 1 to 6, preferably 2 to 4 and more preferably 2 and 3 carbon atoms. Specific examples are CF₃CF₂CF₂CF₂O, CF₃CF₂CF₂O and CF₃CF₂O.

The above x, y, z and n are each preferably 1 to 70, more preferably 1 to 50 and particularly preferably 1 to 30.

It is possible to prepare a triflate or trifluoroacetate of a fluoropolyether by reacting a trifluoromethanesulfonic anhydride or trifluoroacetic anhydride with a fluoropolyether having a hydroxyl group. The reaction is preferably conducted by using one to three equivalents of trifluoromethanesulfonic anhydride or trifluoroacetic anhydride per equivalent of a hydroxyl group, in the presence of a base, at an atmosphere of an inert gas and at room temperature for 1 to 48 hours with stirring. Examples of bases are pyridine, imidazole and tripropylamine. Examples of inert gas are nitrogen and argon.

The triflate is at least one selected among:
A-(CF₂CF₂O)x(CF₂O)yCF₂CH₂OTf, TfOCH₂CF₂O(CF₂CF₂O)x(CF₂O)yCF₂CH₂OTf,
A-(CF₂CF₂CF₂O)zCF₂CF₂CH₂OTf, TfOCH₂CF₂CF₂O(CF₂CF₂CF₂O)zCF₂CF₂CH₂OTf,
A-(CF₂CF₂CF₂CF₂O)nCF₂CF₂CF₂CH₂OTf and TfOCH₂CF₂CF₂CF₂O(CF₂CF₂CF₂CF₂O)nCF₂CF₂CF₂CH₂OTf,
A is fluoroalkoxy, Tf is CF₃SO₂, x, y, z and n are the same as above.

The trifluoroacetate is at least one selected among:
A-(CF₂CF₂O)x(CF₂O)yCF₂CH₂OTfa, TfaOCH₂CF₂O(CF₂CF₂O)x(CF₂O)yCF₂CH₂OTfa,
A-(CF₂CF₂CF₂O)zCF₂CF₂CH₂OTfa, TfaOCH₂CF₂CF₂O(CF₂CF₂CF₂O)zCF₂CF₂CH₂OTfa,
A-(CF₂CF₂CF₂CF₂O)nCF₂CF₂CF₂CH₂OTfa and TfaOCH₂CF₂CF₂CF₂O(CF₂CF₂CF₂CF₂O)nCF₂CF₂CF₂CH₂OTfa,
A is fluoroalkoxy, Tfa is CF₃CO, x, y, z and n are the same as above.

The resulting triflate or trifluoroacetate of the fluoropolyether is decomposed with heating in the presence of a Lewis acid to prepare a mixture of low-molecular weight fluoropolyether and acid fluoride. Examples of the Lewis acids are a metal oxide such as aluminum oxide, and a metal halide such as aluminum chloride. The reaction is preferably conducted by using 0.1 to 30 % by weight of Lewis acid based on the triflate or trifluoroacetate of the fluoropolyether in an atmosphere of an inert gas and at 150 to 350 °C for 1 to 48 hours with heat and stirring.

The acid fluoride is at least one selected among:
A-(CF₂CF₂O)xa(CF₂O)ya-CF₂COF, FOC-CF₂O(CF₂CF₂O)xa(CF₂O)ya-CF₂COF,
A-(CF₂CF₂CF₂O)za-CF₂CF₂COF, FOC-CF₂CF₂O(CF₂CF₂CF₂O)za-CF₂CF₂COF,
A-(CF₂CF₂CF₂CF₂O)na-CF₂CF₂CF₂COF and FOC-CF₂CF₂CF₂O(CF₂CF₂CF₂CF₂O)na-CF₂CF₂CF₂COF,
A is fluoroalkoxy, 0.1x≦xa≦0.9x, 0.1y≦ya≦0.9y, 0.1z≦za≦0.9z, 0.1n≦na≦0.9n, x, y, z and n are the same as above.

Preferable are 0.2x≦xa≦0.8x, 0.2y≦ya≦0.8y, 0.2z≦za≦0.8z, and 0.2n≦na≦0.8n. More preferable are 0.3x≦xa≦0.6x, 0.3y≦ya≦0.6y, 0.3z≦za≦0.6z, and 0.3n≦na≦0.6n.

In the present invention, a fluoropolyether having a molecular weight lower than a starting fluoropolyether is obtained other than the acid fluoride. The fluoropolyether is at least one selected among:
A-(CF₂CF₂O) xa (CF₂O)ya-B,
A-(CF₂CF₂CF₂O)za-B and
A-(CF₂CF₂CF₂CF₂O)na-B,
A is fluoroalkoxy, B is fluoroalkyl, xa, ya, za and na are the same as above.

Examples of the fluoroalkoxy A are as above and those having 1 to 6, preferably 2 to 4 and further preferably 2 and 3 carbon atoms. Specific examples are CF₃CF₂CF₂CF₂O, CF₃CF₂CF₂O and CF₃CF₂O.

Examples of the fluoroalkyl B are those having 1 to 6, preferably 2 to 4 and further preferably 2 and 3 carbon atoms. Specific examples are -CF₂CF₂CF₂CF₃, -CF₂CF₂CF₃ and -CF₂CF₃.

By reacting a reducing agent such as NaBH₄ or LiAlH₄ with the mixture of low-molecular weight fluoropolyether and acid fluoride of the present invention (hereinafter referred to as "the present compound), the acid fluoride is modified to a hydroxyl compound. Further, this hydroxyl compound can be converted to a phosphazene compound such as Patent Literature 4 or a compound containing an aromatic ring such as Patent Literature 5.

Patent Literature 4: US 2010/136371A

Patent Literature 5: JP 2009270093A

The above hydroxyl compound derived from the acid fluoride, the phosphazene compound such as Patent Literature 4 or the compound containing an aromatic ring such as Patent Literature 5 (hereinafter referred to as "the compound derived from the present compound) can be used for example as a lubricant for a magnetic disk.

The compound derived from the present compound is applied to the magnetic disk surface preferably by diluting the compound with a solvent and coating the disk surface with the diluted compound. Examples of useful solvents are PF-5060, PF-5080, HFE-7100 and HFE-7200 manufactured by 3M, Vertrel-XF, product of DuPont, etc.

While the compound derived from the present compound is usable singly, the compound can be used also as mixed in a desired ratio with another material, such as Fomblin Zdol, Ztetraol, Zdol TX, AM manufactured by Solvay Solexis, Demnum manufactured by Daikin Industries, Ltd., Krytox manufactured by DuPont, or the like.

Examples of the hydroxyl compound derived from the acid fluoride are
A-(CF₂CF₂O)xa(CF₂O)ya-CF₂CH₂OH, HO-CH₂CF₂O(CF₂CF₂O)xa(CF₂O)ya-CF₂CH₂OH,
A-(CF₂CF₂CF₂O)za-CF₂CF₂CH₂OH, HO-CH₂CF₂CF₂O(CF₂CF₂CF₂O)za-CF₂CF₂CH₂OH,
A-(CF₂CF₂CF₂CF₂O)na-CF₂CF₂CF₂CH₂OH and HO-CH₂CF₂CF₂CF₂O(CF₂CF₂CF₂CF₂O)na-CF₂CF₂CF₂CH₂OH,
A is fluoroalkoxy, 0.1x≦xa≦0.9x, 0.1y≦ya≦0.9y, 0.1z≦za≦0.9z, 0.1n≦na≦0.9n, x, y, z and n are the same as above.

The phosphazene compound of Patent Literature 4 is shown by the formula below

D-(CH₂-Rf-CH₂O-E-O)n-CH₂-Rf-CH₂-D

wherein n is an integer of 1 to 4, D is a group of the formula (a), E is a group of the formula (b), p is 1 or 2, R is fluoroalkyl having 1 to 4 carbon atoms, Rf is -CF₂O(CF₂CF₂O)x'(CF₂O)y'CF₂- or -CF₂CF₂O(CF₂CF₂CF₂O)z'CF₂CF₂-, x', y' and z' are each a real number of 0 to 50.

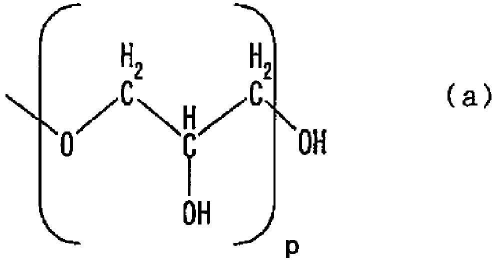

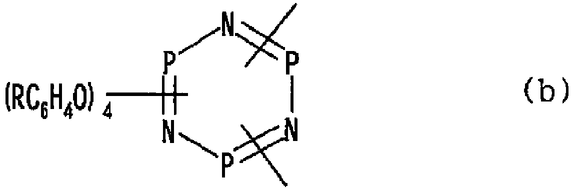

The compound containing an aromatic ring of Patent Literature 5 is shown by the formula below

[HO-(CH₂-R²-CH_{2O}-G-O)ₙ-CH₂-R²-CH₂-O-CH₂]ₚ-R¹

wherein n is an integer of 0 to 6, G is a group of the formula -CH₂CH(OH)CH₂-, R¹ is an aromatic group of C₆H₆₋ₚ, C₆H_{5-q}-O-C₆H₅₋ᵣ or C₁₀H₈₋ₚ, p is an integer of 3 to 6, q and r are each an integer of at least 0, p=q+r, R² is -CF₂₀ (CF₂CF₂O)_{x"}(CF₂O)_{y"}CF₂- or -CF₂CF₂O(CF₂CF₂CF₂O)_{z"}CF₂CF₂-, x" and y" are each a real number of 0 to 30, z" is a real number of 1 to 30.

### EXAMPLES

The invention will be described in more detail with reference to the following examples to which, however, the invention is not limited.

### Example 1

### Preparation of A- (CF₂CF₂CF₂O)zCF₂CF₂CH₂OTf (Compound 1)

### A=CF₃CF₂CF₂O-, Tf=CF₃SO₂-

In an argon atmosphere, 100 g of trifluoromethanesulfonic anhydride was added dropwise for one hour to a mixture of 570 g of dichloromethane, 285g of a fluoropolyether of the formula CF₃CF₂CF₂O(CF₂CF₂CF₂O)zCF₂CF₂CH₂OH (2004 in number average molecular weight and 1.25 in molecular weight distribution) and 34 g of pyridine. The mixture was stirred at room temperature for 17 hours. The mixture was thereafter washed with water, dewatered and purified by silica gel column chromatography, affording 282 g of Compound 1.

Compound 1 was a colorless transparent liquid and 1.74 g/cm³ in density at 20°C. Compound 1 was identified by NMR with the result shown. Compound 1 was 2158 in number average molecular weight which was obtained by a method for determining terminal group using ¹⁹F-NMR (same as hereinafter). ¹⁹F-NMR (solvent: none, reference material: OCF₂CF₂CF₂O in the obtained product being taken as -129.7 ppm)
δ=-129.7ppm
[20F, -OCF₂CF₂CF₂O-]
δ=-83.7ppm
[40F, -OCF₂CF₂CF₂O-]
δ=-124.7ppm
[2F, -OCF₂CF₂CH₂OSO₂CF₃]
δ=-86.8ppm
[2F, -OCF₂CF₂CH₂OSO₂CF₃]
δ=-76.0ppm
[₃F, -OCF₂CF₂CH₂OSO₂CF₃]
δ=-130.7ppm
[2F, CF₃CF₂CF₂O-]
δ=-84.7ppm
[2F, CF₃CF₂CF₂O-]
δ=-82.4ppm
[3F, CF₃CF₂CF₂O-]
z=10.2
¹H-NMR (solvent: none, reference material: D₂O)
δ = 3.2ppm
[2H, -OCF₂CF₂CH₂OSO₂CF₃]

### Example 2

### Preparation of a mixture (Mixture 1) of CF₃CF₂O(CF₂CF₂CF₂O)_{za}CF2CF2COF (Compound 2), FOCCF₂CF₂O(CF₂CF₂CF₂O)_{za}CF₂CF₂COF (Compound 3) and CF₃CF₂CF₂O(CF₂CF₂CF₂O)_{za}CF₂CF₂CF₃ (Compound 4)

In an argon atmosphere, 57 g of Compound 1 (2158 in number average molecular weight and 1.23 in molecular weight distribution) and 3 g of aluminum chloride were heated for reflux at 250 °C for 2 hours. After cooled to room temperature, solid reaction byproducts were filtered off to obtain 28 g of Mixture 1 of Compound 2, Compound 3 and Compound 4 as a filtrate.

Mixture 1 was a light yellow transparent liquid and 1.69 g/cm³ in density at 20°C. Mixture 1 was identified by NMR with the result shown. Mixture 1 was 1045 in number average molecular weight.
¹⁹F-NMR (solvent: none, reference material: OCF₂CF₂CF₂O in the obtained product being taken as -129.7 ppm)
δ=-129.7ppm
[9F, -OCF₂CF₂CF₂O-]
δ=-83.7ppm
[17F, -OCF₂CF₂CF₂O-]
δ=-86.3ppm
[2F, -OCF₂CF₂COF]
δ=-122.1ppm
[2F, -OCF₂CF₂COF]
δ=-21.7ppm
[1F, -OCF₂CF₃COF]
δ=-130.7ppm
[2F, CF₃CF₂CF₂O-]
δ=-84.7ppm
[2F, CF₃CF₂CF₂O-]
δ=-82.4ppm
[3F, CF₃CF₂CF₂O-]
za=4.28
Example 3

### Preparation of TfaOCH₂CF₂O(CF₂CF₂O)ₓ(CF₂O)_{y}CF₂CH₂OTfa (Compound 5)

### Tfa=CF₃CO-

In an argon atmosphere, 50 g of trifluoroacetic anhydride was added dropwise for one hour to 200g of a fluoropolyether of the formula

HOCH₂CF₂O(CF₂CF₂O)x(CF₂O)yCF₂CH₂OH (2071 in number average molecular weight and 1.15 in molecular weight distribution). The mixture was stirred at room temperature for 17 hours. Thereafter, low-boiling components were removed by an evaporator, affording 202 g of Compound 5.

Compound 5 was a colorless transparent liquid and 1.69 g/cm³ in density at 20°C. Compound 5 was identified by NMR with the result shown. Compound 5 was 2265 in number average molecular weight.
¹⁹F-NMR (solvent: none, reference material: OCF₂CF₂CF₂CF₂O in the obtained product being taken as -125.8 ppm)
δ=-52.1ppm, -53.7ppm, -55.4ppm
[21F, -OCF₂O-]
δ=-78.7ppm, -80.6ppm
[4F, -CF₂CH₂OCOCF₃]
δ=-76.7ppm
[6F, -CF₂CH₂OCOCF₃]
δ=-89.1ppm, -90.7ppm
[40F, -OCF₂CF₂O-]
x=10.1, y=10.4
¹H-NMR (solvent: none, reference material: D₂O)
δ = 5.0ppm
[4H, -OCF₂CH₂OCOCF₃]

### Example 4

### Preparation of a mixture (Mixture 2) of CF₃CF₂O(CF₂CF₂O)ₓₐ(CF₂O)_{ya}CF₂COF (Compound 6), FOCCF₂O(CF₂CF₂O)ₓₐ(CF₂O)_{ya}CF₂COF (Compound 7) and CF₃CF₂O(CF₂CF₂O)ₓₐ(CF₂O)_{ya}CF₂CF₃ (Compound 8)

In an argon atmosphere, 60 g of Compound 5 (2265 in number average molecular weight and 1.14 in molecular weight distribution) and 6 g of aluminum oxide were heated for reflux at 250 °C for 2 hours. After cooled to room temperature, solid reaction byproducts were filtered off to obtain 30 g of Mixture 2 of Compound 6, Compound 7 and Compound 8 as a filtrate.

Mixture 2 was a light yellow transparent liquid and 1.70 g/cm³ in density at 20°C. Mixture 2 was identified by NMR with the result shown. Mixture 2 was 968 in number average molecular weight.
¹⁹F-NMR (solvent: none, reference material: OCF₂CF₂CF₂O in the obtained product being taken as -125.8 ppm)
δ=-52.1ppm, -53.7ppm, -55.4ppm
[8F, -OCF₂O-],
δ=-78.7ppm, -80.6ppm
[2F, -CF₂CF₃],
δ=-76.7ppm
[3F, -CF₂CF₃],
δ=-89.1ppm, -90.7ppm
[16.4F, -OCF₂CF₂O-]
za=4.10, ya=4.18

### Reference Example 1

### Preparation of a mixture (Mixture 3) of CF₃CF₂O(CF₂CF₂CF₂O)_{za}CF₂CF₂CH₂OH (Compound 9), HOCH₂CF₂CF₂O(CF₂CF₂CF₂O)_{za}CF₂CF₂CH₂OH (Compound 10) and CF₃CF₂CF₂O(CF₂CF₂CF₂O)_{za}CF₂CF₂CF₃ (Compound 11)

In an argon atmosphere, 10 ml of dehydrated THF is mixed with 0.6 g of LiAlH₄ and ice-cooled. To a mixture was added dropwise a solution of 3 g of Mixture 1 (1045 in number average molecular weight) obtained in Example 2 dissolved in HFE-7100. After the addition, the mixture was stirred for one night at room temperature. To the mixture was added methanol and excess of LiAlH₄ was deactivated, then washed with acid and washed with water. The mixture was dried over dehydrating agent, and the agent was filtered off. The filtrate was concentrated to give 2.4 g of Mixture 3 containing Compound 9, Compound 10 and Compound 11.

Mixture 3 was a light yellow transparent liquid and 1.65 g/cm³ in density at 20°C. Mixture 3 was identified by NMR with the result shown. Mixture 3 was 981 in number average molecular weight.
¹⁹F-NMR (solvent: none, reference material: OCF₂CF₂CF₂O in the obtained product being taken as -129.7 ppm)
δ=-129.7ppm
[8F,-OCF₂CF₂CF₂O-]
δ=-83.7ppm
[16F, -OCF₂CF₂CF₂O-]
δ=-86.4ppm
[2F,-OCF₂CF₂CH₂OH]
δ=-127.5ppm
[2F,-OCF₂CF₂CH₂OH]
δ=-130.7ppm
[2F, CF₃CF₂CF₂O-]
δ=-84.7ppm
[2F, CF₃CF₂CF₂O-]
δ=-82.4ppm
[3F, CF₃CF₂CF₂O-]
za=4.01
¹H-NMR (solvent: none, reference material: D₂O)
δ=4.0ppm
[2H,-OCF₂CF₂CH₂OH]
δ=4.3ppm
[1H,-OCF₂CF₂CH₂OH]

### INDUSTRIAL APPLICABILITY

In the present invention, it is possible to prepare a mixture of fluoropolyether having a molecular weight lower than a starting fluoropolyether and acid fluoride by modifying a hydroxyl group of the starting fluoropolyether to a triflate or trifluoroacetate and cleaving the fluoropolyether with heating in the presence of a Lewis acid.

## Claims

1. A process for preparing a mixture of low-molecular weight fluoropolyether and acid fluoride by decomposing with heating a triflate of a fluoropolyether having a hydroxyl group in the presence of a Lewis acid, wherein the triflate is at least one selected among
A-(CF₂CF₂O)x(CF₂O)yCF₂CH₂OTf
TfOCH₂CF₂O(CF₂CF₂O)x(CF₂O)yCF₂CH₂OTf
A-(CF₂CF₂CF₂O)zCF₂CF₂CH₂OTf
TfOCH₂CF₂CF₂O(CF₂CF₂CF₂O)zCF₂CF₂CH₂OTf
A-(CF₂CF₂CF₂CF₂O)nCF₂CF₂CF₂CH₂OTf
TfOCH₂CF₂CF₂CF₂O(CF₂CF₂CF₂CF₂O)nCF₂CF₂CF₂CH₂OTf,
the acid fluoride is at least one selected among
A-(CF₂CF₂O) xa (CF₂O)ya-CF₂COF
FOC-CF₂O(CF₂CF₂O)xa(CF₂O)ya-CF₂COF
A-(CF₂CF₂CF₂O)za-CF₂CF₂COF
FOC-CF₂CF₂O(CF₂CF₂CF₂O)za-CF₂CF₂COF
A-(CF₂CF₂CF₂CF₂O)na-CF₂CF₂CF₂COF
FOC-CF₂CF₂CF₂O(CF₂CF₂CF₂CF₂O)na-CF₂CF₂CF₂COF, and
the low-molecular weight fluoropolyether is at least one selected among
A-(CF₂CF₂O)xa(CF₂O)ya-B,
A-(CF₂CF₂CF₂O)za-B and
A-(CF₂CF₂CF₂CF₂O)na-B,
A is fluoroalkoxy, B is fluoroalkyl, Tf is CF₃SO₂, x, y, z and n are a real number of 1 to 100, 0.1x≦xa≦0.9x, 0.1y≦ya≦0.9y, 0.1z≦za≦0.9z, 0.1n≦na≦0.9n.

2. A process for preparing a mixture of low-molecular weight fluoropolyether and acid fluoride by decomposing with heating a trifluoroacetate of a fluoropolyether having a hydroxyl group in the presence of a Lewis acid, wherein the trifluoroacetate is at least one selected among
A-(CF₂CF₂O)x(CF₂O)yCF₂CH₂OTfa
TfaOCH₂CF₂O(CF₂CF₂O)x(CF₂O)yCF₂CH₂OTfa
A-(CF₂CF₂CF₂O)zCF₂CF₂CH₂OTfa
TfaOCH₂CF₂CF₂O(CF₂CF₂CF₂O)zCF₂CF₂CH₂OTfa
A-(CF₂CF₂CF₂CF₂O)nCF₂CF₂CF₂CH₂OTfa
TfaOCH₂CF₂CF₂CF₂O(CF₂CF₂CF₂CF₂O)nCF₂CF₂CF₂CH₂OTf a,
the acid fluoride is at least one selected among
A-(CF₂CF₂O)xa(CF₂O)ya-CF₂COF
FOC-CF₂O(CF₂CF₂O)xa(CF₂O)ya-CF₂COF
A-(CF₂CF₂CF₂O)za-CF₂CF₂COF
FOC-CF₂CF₂O(CF₂CF₂CF₂O)za-CF₂CF₂COF
A-(CF₂CF₂CF₂CF₂O)na-CF₂CF₂CF₂COF
FOC-CF₂CF₂CF₂O(CF₂CF₂CF₂CF₂O)na-CF₂CF₂CF₂COF, and
the low-molecular weight fluoropolyether is at least one selected among
A-(CF₂CF₂O)xa(CF₂O)ya-B,
A-(CF₂CF₂CF₂O)za-B and
A-(CF₂CF₂CF₂CF₂O)na-B,
A is fluoroalkoxy, B is fluoroalkyl, Tfa is CF₃CO, x, y, z and n are a real number of 1 to 100, 0.1x≦xa≦0.9x, 0.1y≦ya≦ 0.9y, 0.1z≦za≦0.9z, 0.1n≦na≦0.9n.

## Patentansprüche

1. Verfahren zur Herstellung eines Gemisches aus Fluorpolyether niedrigen Molekulargewichts und Säurefluorid durch Zersetzen eines Triflats eines Fluorpolyethers mit einer Hydroxylgruppe unter Erwärmen in Gegenwart einer LewisSäure, wobei das Triflat mindestens eines ist ausgewählt aus
A-(CF₂CF₂O)x(CF₂O)yCF₂CH₂OTf
TfOCH₂CF₂O(CF₂CF₂O)x(CF₂O)yCF₂CH₂OTf
A-(CF₂CF₂CF₂O)zCF₂CF₂CH₂OTf
TfOCH₂CF₂CF₂O(CF₂CF₂CF₂O)zCF₂CF₂CH₂OTf
A-(CF₂CF₂CF₂CF₂O)nCF₂CF₂CF₂CH₂OTf
TfOCH₂CF₂CF₂CF₂O(CF₂CF₂CF₂CF₂O)nCF₂CF₂CF₂CH₂OTf,
das Säurefluorid mindestens eines ist ausgewählt aus
A-(CF₂CF₂O)xa(CF₂O)ya-CF₂COF
FOC-CF₂O(CF₂CF₂O)xa(CF₂O)ya-CF₂COF
A-(CF₂CF₂CF₂O)za-CF₂CF₂COF
FOC-CF₂CF₂O(CF₂CF₂CF₂O)za-CF₂CF₂COF
A-(CF₂CF₂CF₂CF₂O)na-CF₂CF₂CF₂COF
FOC-CF₂CF₂CF₂O(CF₂CF₂CF₂CF₂O)na-CF₂CF₂CF₂COF, und
der Fluorpolyether niedrigen Molekulargewichts mindestens einer ist ausgewählt aus
A-(CF₂CF₂O)xa(CF₂O)ya-B,
A-(CF₂CF₂CF₂O)za-B und
A-(CF₂CF₂CF₂CF₂O)na-B,
A Fluoralkoxy ist, B Fluoralkyl ist, Tf CF₃SO₂ ist, x, y, z und n eine reelle Zahl von 1 bis 100 sind, 0.1x≦xa≦0.9x, 0.1y≦ya≦0.9y, 0.1z≦za≦0.9z, 0.1n≦na≦0.9n.

2. Verfahren zur Herstellung eines Gemisches aus Fluorpolyether niedrigen Molekulargewichts und Säurefluorid durch Zersetzen eines Trifluoracetats eines Fluorpolyethers mit einer Hydroxylgruppe unter Erwärmen in Gegenwart einer LewisSäure, wobei das Trifluoracetat mindestens eines ist ausgewählt aus
A-(CF₂CF₂O)x(CF₂O)yCF₂CH₂OTfa
TfaOCH₂CF₂O(CF₂CF₂O)x(CF₂O)yCF₂CH₂OTfa
A-(CF₂CF₂CF₂O)zCF₂CF₂CH₂OTfa
TfaOCH₂CF₂CF₂O(CF₂CF₂CF₂O)zCF₂CF₂CH₂OTfa
A-(CF₂CF₂CF₂CF₂O)nCF₂CF₂CF₂CH₂OTfa
TfaOCH₂CF₂CF₂CF₂O(CF₂CF₂CF₂CF₂O)nCF₂CF₂CF₂CH₂OTfa,
das Säurefluorid mindestens eines ist ausgewählt aus
A-(CF₂CF₂O)xa(CF₂O)ya-CF₂COF
FOC-CF₂O(CF₂CF₂O)xa(CF₂O)ya-CF₂COF
A-(CF₂CF₂CF₂O)za-CF₂CF₂COF
FOC-CF₂CF₂O(CF₂CF₂CF₂O)za-CF₂CF₂COF
A-(CF₂CF₂CF₂CF₂O)na-CF₂CF₂CF₂COF
FOC-CF₂CF₂CF₂O(CF₂CF₂CF₂CF₂O)na-CF₂CF₂CF₂COF, und
der Fluorpolyether niedrigen Molekulargewichts mindestens einer ist ausgewählt aus
A-(CF₂CF₂O)xa(CF₂O)ya-B,
A-(CF₂CF₂CF₂O)za-B und
A-(CF₂CF₂CF₂CF₂O)na-B,
A Fluoralkoxy ist, B Fluoralkyl ist, Tfa CF₃CO ist, x, y, z und n eine reelle Zahl von 1 bis 100 sind, 0.1x≦xa≦0.9x, 0.1y≦ya≦0.9y, 0.1z≦za≦0.9z, 0.1n≦na≦0.9n.

## Revendications

1. Procédé de préparation d'un mélange de fluoropolyéther de faible masse moléculaire et de fluorure d'acide par décomposition avec chauffage d'un triflate d'un fluoropolyéther ayant un groupe hydroxyle en présence d'un acide de Lewis, dans lequel le triflate est au moins un choisi parmi
A-(CF₂CF₂O)x(CF₂O)yCF₂CH₂OTf
TfOCH₂CF₂O(CF₂CF₂O)x(CF₂O)yCF₂CH₂OTf
A-(CF₂CF₂CF₂O)zCF₂CF₂CH₂OTf
TfOCH₂CF₂CF₂O(CF₂CF₂CF₂O)zCF₂CF₂CH₂OTf
A-(CF₂CF₂CF₂CF₂O)nCF₂CF₂CF₂CH₂OTf
TfOCH₂CF₂CF₂CF₂O(CF₂CF₂CF₂CF₂O)nCF₂CF₂CF₂CH₂OTf,
le fluorure d'acide est au moins un choisi parmi
A-(CF₂CF₂O)xa(CF₂O)ya-CF₂COF
FOC-CF₂O(CF₂CF₂O)xa(CF₂O)ya-CF₂COF
A-(CF₂CF₂CF₂O)za-CF₂CF₂COF
FOC-CF₂CF₂O(CF₂CF₂CF₂O)za-CF₂CF₂COF
A-(CF₂CF₂CF₂CF₂O)na-CF₂CF₂CF₂COF
FOC-CF₂CF₂CF₂O(CF₂CF₂CF₂CF₂O)na-CF₂CF₂CF₂COF, et
le fluoropolyéther de faible masse moléculaire est au moins un choisi parmi
A-(CF₂CF₂O)xa(CF₂O)ya-B,
A-(CF₂CF₂CF₂O)za-B et
A-(CF₂CF₂CF₂CF₂O)na-B,
A est un fluoroalcoxy, B est un fluoroalkyle, Tf est CF₃SO₂, x, y, z et n sont un nombre réel de 1 à 100, 0,1x≤xa≤0,9x, 0,1y≤ya≤0,9y, 0,1z≤za≤0,9z, 0,1n≤na≤0,9n.

2. Procédé de préparation d'un mélange de fluoropolyéther de faible masse moléculaire et de fluorure d'acide par décomposition avec chauffage d'un trifluoroacétate d'un fluoropolyéther ayant un groupe hydroxyle en présence d'un acide de Lewis, dans lequel le trifluoroacétate est au moins un choisi parmi
A-(CF₂CF₂O)x(CF₂O)yCF₂CH₂OTfa
TfaOCH₂CF₂O(CF₂CF₂O)x(CF₂O)yCF₂CH₂OTfa
A-(CF₂CF₂CF₂O)zCF₂CF₂CH₂OTfa
TfaOCH₂CF₂CF₂O(CF₂CF₂CF₂O)zCF₂CF₂CH₂OTfa
A-(CF₂CF₂CF₂CF₂O)nCF₂CF₂CF₂CH₂OTfa
TfaOCH₂CF₂CF₂CF₂O(CF₂CF₂CF₂CF₂O)nCF₂CF₂CF₂CH₂OTfa,
le fluorure d'acide est au moins un choisi parmi
A-(CF₂CF₂O)xa(CF₂O)ya-CF₂COF
FOC-CF₂O(CF₂CF₂O)xa(CF₂O)ya-CF₂COF
A-(CF₂CF₂CF₂O)za-CF₂CF₂COF
FOC-CF₂CF₂O(CF₂CF₂CF₂O)za-CF₂CF₂COF
A-(CF₂CF₂CF₂CF₂O)na-CF₂CF₂CF₂COF
FOC-CF₂CF₂CF₂O(CF₂CF₂CF₂CF₂O)na-CF₂CF₂CF₂COF, et
le fluoropolyéther de faible masse moléculaire est au moins un choisi parmi
A-(CF₂CF₂O)xa(CF₂O)ya-B,
A-(CF₂CF₂CF₂O)za-B et
A-(CF₂CF₂CF₂CF₂O)na-B,
A est un fluoroalcoxy, B est un fluoroalkyle, Tfa est CF₃CO, x, y, z et n sont un nombre réel de 1 à 100, 0,1x≤xa≤0,9x, 0,1y≤ya≤0,9y, 0,1z≤za≤0,9z, 0,1n≤na≤0,9n.
